Europäisches Patentamt

European Patent Office

Office européen des brevets

(19)

(11) Veröffentlichungsnummer: **0 231 844**
**B1**

# EUROPÄISCHE PATENTSCHRIFT

(12)

(45) Veröffentlichungstag der Patentschrift:
03.01.90

(51) Int. Cl.⁴: **A61K 7/06**

(21) Anmeldenummer: **87101003.9**

(22) Anmeldetag: **24.01.87**

(54) Haarkosmetische Mittel.

(30) Priorität: **06.02.86 DE 3603595**

(43) Veröffentlichungstag der Anmeldung:
**12.08.87 Patentblatt 87/33**

(45) Bekanntmachung des Hinweises auf die Patenterteilung:
**03.01.90 Patentblatt 90/1**

(84) Benannte Vertragsstaaten:
**AT BE CH DE ES FR GB GR IT LI NL SE**

(56) Entgegenhaltungen:
EP-A- 0 102 534
DE-A- 3 233 388
FR-A- 1 337 769

**Aerztliche Kosmetologie 17, 91-110 (1987)**

**Die Akte enthält technische Angaben, die nach dem Eingang der Anmeldung eingereicht wurden und die nicht in dieser Patentschrift enthalten sind.**

(73) Patentinhaber: **Beiersdorf Aktiengesellschaft,
Unnastrasse 48, D-2000 Hamburg 20(DE)**

(72) Erfinder: **Engel, Walter, Diesterwegstrasse 22,
D-2080 Pinneberg(DE)**
Erfinder: **Hoppe, Udo, Dr., Lottbeker Weg 7,
D-2000 Hamburg 65(DE)**

## Beschreibung

Die Erfindung betrifft haarkosmetische Mittel auf wäßriger Basis, welche neben üblichen Bestandteilen Sericin und Pelargonsäure als Wirkstoffe enthalten. Haarkosmetische Mittel wirken auf Haare, Kopfhaut und Frisur. Sie umfassen

(1) Mittel zur Reinigung und Pflege (z.B. Shampoos, Haarwasser, Haarlotionen, Frisierhilfsmittel, Haarkurmittel),
(2) Mittel zur Verschönerung und Erhaltung der Frisur (z.B. Haarfestiger, Haarspray, Dauerwellmittel),
(3) Mittel zur Verschönerung der Haarfarbe (z.B. Haartönungen, Haarfärbemittel, Blondiermittel).

Haarkosmetische Mittel auf wäßriger Basis mit einem Gehalt an Sericin, einem Protein, das z.B. bei der Entbastung von Rohseide gewonnen wird, sind an sich bereits aus der DE-OS 32 33 388 bekannt. Darin wird als Vorzug derartiger Mittel ein starker Hautglättungseffekt und ein substantives Verhalten zu menschlichem Haar, das Cuticuläschäden in der Haaroberfläche verringert, beschrieben. Gemäß der DE-OS 32 33 388 werden die haarkosmetischen Mittel vorzugsweise auf einen neutralen bis sauren pH-Wert eingestellt.

Es wurde jedoch festgestellt, daß die bisher üblichen, marktgängigen "alkalifreien", "neutralen" oder "sauren" Shampoos keine oder eine zu geringe Pufferkapazität aufweisen. D.h. bei bestimmungsgemäßer Anwendung liegt der pH-Wert derartiger Shampoos, dann zur Waschflotte verdünnt, im pH-Bereich des Wassers, der in der Regel zwischen 6,5 und 9,5 liegt. Die angestrebte Wirkung durch pH-Wert-Stabilisierung im physiologischen Bereich (pH-Wert 5,7 bis 5,9), nämlich

(1) Ein Minimum an Quellung des Keratinmaterials und
(2) dadurch die Cuticulaschuppenschicht wieder fester an den Haarschaft zu legen,

wird daher so nicht erreicht. Die üblicherweise eingesetzten Säurekomponenten werden durch die Wasserinhaltsstoffe neutral gestellt und ausgespült.

Mittelkettige Fettsäuren ($C_8$ bis $C_{12}$), vorzugsweise die Dekansäure (Caprinsäure) oder die Dodekansäure (Laurinsäure) wurden bereits in der DE-OS 28 28 352 als Mittel zur Behandlung seborrhoischer Zustände vorgeschlagen. Lösungen wirksamer Konzentrationen derartiger Säuren wurden jedoch nur in Alkoholen bzw. in stark alkoholhaltigen Lösungsmittelgemischen erhalten.

Demgemäß bestand die Aufgabe der Erfindung darin, ein mildes, besonders verträgliches haarkosmetisches Mittel auf wäßriger Basis zu entwickeln, das unter Beibehaltung der guten haut- und haarpflegenden Eigenschaften eines sericinhaltigen Mittels zugleich auch den pH-Wert auf der Kopfhaut und den Haaren im physiologischen Bereich stabilisiert, um die Störung der natürlichen Verhältnisse an Kopfhaut und Haaren bei der Anwendung derartiger Mittel möglichst gering zu halten. Dies insbesondere im Hinblick auf eine Anwendung in Ergänzung zu therapeutischen Maßnahmen, z.B. Intervallbehandlung gegen Pilz- oder Bakterienbefall.

Es wurde nun überraschenderweise gefunden, daß sich Pelargonsäure (Nonansäure) im Zusammenwirken mit Sericin (möglicherweise unter Komplexbildung) auch als wäßrige Zubereitung einsetzen läßt. Bei Anwendung eines derartigen haarkosmetischen Mittels auf wäßriger Basis wird eine Stabilisierung des pH-Wertes im physiologischen Bereich bewirkt. Zugleich wird auch bei längerer Anwendung keine Schuppenbildung induziert, vielmehr tritt bei einigen Formen der Kopfschuppen sogar eine Reduzierung ein.

Gegenstand der Erfindung ist daher ein haarkosmetisches Mittel auf wäßriger Basis, vorzugsweise in Form eines Haarkurmittels, besonders bevorzugt in Form eines Haarwaschmittels (Shampoos), das dadurch gekennzeichnet ist, daß es neben üblichen Bestandteilen Sericin und Pelargonsäure enthält. Dabei hat sich ein Anteil von 0,01 bis 1,0 Gew.-%, vorzugsweise 0,02 bis 0,6 Gew.-% Sericin und 0,01 bis 6,0 Gew.-% vorzugsweise 0,01 bis 1,0 Gew.-%, besonders bevorzugt 0,03 bis 0,5 Gew.-% Pelargonsäure als besonders geeignet erwiesen.

Zur Erzielung einer optimalen Wirkung wird ein derartiges haarkosmetisches Mittel in an sich bekannter Weise durch Zusatz einer geeigneten Säure, z.B. Zitronensäure, Ethercarbonsäuren, Alkyletherphosphorsäuren oder eines sauren Puffersystems,z.B. Zitronensäure/Zitrat-Puffer, auf einen sauren pH-Wert (pH: 5 - 6) eingestellt.

Es konnte nachgewiesen werden, daß bei einem erfindungsgemäßen Haarwaschmittel mit Sericin und Pelargonsäure im Vergleich zu einem handelsüblichen sauren Shampoo ohne diesen Zusatz auch bei Verdünnung mit größeren Mengen Wasser der pH-Wert weniger stark ansteigt, also eine geringere Alkalisierung eintritt. Dieser Effekt wird bei üblichen Anwendungskonzentrationen auch auf dem Haar wirksam, so daß die unerwünschte in wässrigem Medium auftretende Quellung von Haaren und Haarböden vermieden wird. Ohne Pelargonsäure-Zusatz ist dagegen bereits eine Quellung des Haares feststellbar.

Zugleich wird der Schuppenstatus positiv beeinflußt: Während bei einem Haarwaschmittel ohne Pelargonsäure die Schuppenbildung in den ersten zwei Wochen der Anwendung zwar abnimmt, dann aber - teilweise sogar unter Bildung größerer Schuppen - wieder zunehmen kann, wird bei einem erfindungsgemäßen Haarwaschmittel nach der gleichfalls zu beobachtenden Abnahme der Schuppenbildung im weiteren Verlauf ein nahezu gleichbleibender Schuppenstatus festgestellt.

Die haarkosmetischen Mittel gemäß der Erfindung können außerdem in Anpassung an den jeweils vorgesehenen Verwendungszweck in derartigen Mitteln übliche Komponenten in den üblichen Mengenverhältnissen enthalten, wie waschaktive Substanzen (Tenside) der verschiedensten Art (sowohl anionenaktive, kationenaktive, nicht-ionische Verbindungen als auch amphotere Verbindungen), emulgierend wirkende Bestandteile, Viskositätsregler, hydrophobierende Substanzen (z.B. Wachse, Harze oder Silicone), Stabilisatoren, außerdem ggf. Perglanzmittel, Antioxydantien, UV-Absorber, Konservierungsmittel, Farbstoffe und Parfüms sowie weitere übliche Rezeptur-Bestandteile.

Die Herstellung erfolgt in an sich bekannter Weise durch einfaches Vermischen der Bestandteile unter Rühren. Die Reihenfolge der Zugabe der Komponenten ist im Prinzip beliebig, in einer bevorzugten Verfahrensweise werden jedoch Pelargonsäure und Parfüm als letztes eingearbeitet, nachdem der gewünschte pH-Wert bereits mit Hilfe der Säure bzw. des sauren Puffersystems eingestellt wurde.

### Beispiel 1 (Shampoo)

| | |
|---|---|
| Triisopropanolaminlaurylethersulfat 40%ig | 42,0 g |
| Fettsäureamidoalkylbetain 30%ig (Tego Betain L7, Goldschmidt) | 12,0 g |
| Fettalkoholpolyglykolethercarbonsäure (Akypo RLM 150, Chem-Y-GmbH) | 2,0 g |
| Sericin | 0,4 g |
| Pelargonsäure | 0,4 g |
| Wasser, Konservierungsstoffe, Parfüm | auf 100 g |

Die Viskosität kann durch Zugabe von NaCl nach Wunsch eingestellt werden.

### Beispiel 2 (Shampoo)

| | |
|---|---|
| Triisopropanolaminlaurylethersulfat 40%ig | 30,0 g |
| Fettsäureamidoalkylbetain 30%ig | 5,0 g |
| Fettsäureamidoalkyldimethylaminoxid 35%ig (Aminoxid WS35, Goldschmidt) | 3,0 g |
| Fettalkoholpolyglykolethercarbonsäure | 1,0 g |
| Kokosfettsäurediethanolamid | 2,0 g |
| Sericin | 0,6 g |
| Pelargonsäure | 0,5 g |
| Wasser, Konservierungsstoffe, Parfüm | auf 100 g |

Die Viskosität kann durch Zugabe von NaCl nach Wunsch eingestellt werden.

Beispiel 3 (Shampoo)

| | |
|---|---|
| Natriumlaurylethersulfat 27,5%ig | 25,0 g |
| Eiweiß-Fettsäurekondensat-Kalium-salz 30%ig (Lamepon S, Grünau) | 30,0 g |
| Polypeptid-Lösung 34%ig (Nutrilan L, Grünau) | 5,0 g |
| Alkylpolyglykoletherphosphat (Phospatal 201, Zschimmer & Schwarz) | 1,0 g |
| Sericin | 0,3 g |
| Pelargonsäure | 0,05 g |
| Wasser, Konservierungsstoffe, Parfüm | auf 100 g |

Die Viskosität kann durch Zugabe von NaCl nach Wunsch eingestellt werden.

Beispiel 4 (Shampoo)

| | |
|---|---|
| Triethanolaminlaurylsulfat 48%ig | 15,0 g |
| Cocosamphoglycinat 30%ig (Dehyton G, Henkel) | 8,0 g |
| Kokosfettsäurediethanolamid | 3,0 g |
| Alkylpolyglykoletherphosphat | 2,0 g |
| Sericin | 0,5 g |
| Pelargonsäure | 0,3 g |
| Wasser, Konservierungsstoffe, Parfüm | auf 100 g |

Die Viskosität kann durch Zugabe von NaCl nach Wunsch eingestellt werden.

Beispiel 5 (Shampoo)

| | |
|---|---|
| Fettsäureamidoalkylbetain 30%ig | 10,0 g |
| Triethanolaminlaurylsulfat 48%ig | 30,0 g |
| Kokosfettsäurediethanolamid | 2,0 g |
| Zitronensäure | 1,0 g |
| Sericin | 0,3 g |
| Pelargonsäure | 0,06 g |
| Wasser, Konservierungsstoffe, Parfüm | auf 100 g |

Die Viskosität kann durch Zugabe von NaCl nach Wunsch eingestellt werden.

Beispiel 6 (Perlshampoo)

| | |
|---|---|
| Triisopropanolaminlaurylethersulfat 40%ig | 40,0 g |
| Fettsäureamidoalkylbetain 30%ig | 15,0 g |
| Fettalkoholpolyglykolethercarbonsäure | 1,0 g |
| Kokosfettsäurediethanolamid | 4,0 g |
| Perlglanzmittel | 4,0 g |
| Sericin | 0,4 g |
| Pelargonsäure | 0,07 g |
| Wasser, Konservierungsmittel, Parfüm | auf 100 g |

Die Viskosität kann durch Zugabe von NaCl nach Wunsch eingestellt werden.

Beispiel 7 (Perlshampoo)

| | |
|---|---|
| Triisopropanolaminlaurylethersulfat 40%ig | 42,0 g |
| Fettsäureamidoalkylbetain 30%ig | 15,0 g |
| Kokosfettsäurediethanolamid | 4,0 g |
| Polypeptid-Lösung 34%ig | 2,0 g |
| Alkylpolyglykoletherphosphat | 1,0 g |
| Perlglanzmittel | 3,0 g |
| Sericin | 0,4 g |
| Pelargonsäure | 0,08 g |
| Wasser, Konservierungstmittel, Parfüm | auf 100 g |

Die Viskosität kann durch Zugabe von NaCl nach Wunsch eingestellt werden.

Beispiel 8 (Perlshampoo)

| | |
|---|---|
| Triisopropanolaminlaurylethersulfat 40%ig | 45,0 g |
| Fettalkoholpolyglykolethercarbonsäure | 2,0 g |
| Kokosfettsäurediethanolamid | 5,0 g |
| Perlglanzmittel | 3,0 g |
| Sericin | 0,5 g |
| Pelargonsäure | 0,1 g |
| Wasser, Konservierungsmittel, Parfüm | auf 100 g |

Die Viskosität kann durch Zugabe von NaCl nach Wunsch eingestellt werden.

Beispiel 9 (Haarkur-Packung)

| | |
|---|---|
| Cetylstearylalkohol (Emulgade F, Henkel) | 8,0 g |
| Wollwachsalkohol | 0,5 g |
| Cetylalkohol | 0,6 g |
| Lanolin | 0,5 g |
| Laurylpyridiniumchlorid | 0,5 g |
| Methylpolysiloxan (Baysilon M300, Bayer) | 0,2 g |
| Sorbitol | 5,0 g |
| Zitronensäure | 0,5 g |
| Sericin | 0,4 g |
| Pelargonsäure | 0,1 g |
| Wasser, Parfüm, Konservierungsmittel | auf 100 g |

Beispiel 10 (Haarkur-Emulsion)

| | |
|---|---|
| Cetylstearylalkohol | 3,0 g |
| Wollwachsalkohol | 0,5 g |
| Cetylalkohol | 0,5 g |
| Lanolin | 0,5 g |
| Laurylpyridiniumchlorid | 0,5 g |
| Methylpolysiloxan | 0,2 g |
| Sorbitol | 5,0 g |
| Zitronensäure | 0,4 g |
| Sericin | 0,6 g |
| Pelargonsäure | 0,5 g |
| Wasser, Konservierungsmittel, Parfüm | auf 100 g |

Beispiel 11 (Haarkur-Creme-Rinse)

| | |
|---|---|
| Glycerinmonodistearat (Teginacid R, Goldschmidt) | 3,0 g |
| Cetylalkohol | 0,5 g |
| Ölsäuredecylester | 5,0 g |
| Isopropylmyristat | 2,5 g |
| Dialkyldimethylammoniumchlorid (Genamin DSAC, Hoechst) | 2,0 g |
| Sorbitol | 3,0 g |
| Proteinhydrolysat | 2,0 g |
| Propylenglykol | 1,5 g |
| Zitronensäure | 0,3 g |
| Sericin | 0,3 g |
| Pelargonsäure | 0,07 g |
| Wasser, Konservierungsmittel, Parfüm | auf 100 g |

Beispiel 12 (Haarkur-Rinse)

| | |
|---|---|
| Cetylalkohol | 1,0 g |
| Dialkyldimethylammoniumchlorid | 2,0 g |
| Sorbitol | 1,0 g |
| Zitronensäure | 0,3 g |
| Sericin | 0,4 g |
| Pelargonsäure | 0,04 g |
| Wasser, Konservierungsmittel, Parfüm | auf 100 g |

**Patentansprüche**

1. Haarkosmetische Mittel auf wäßriger Basis, dadurch gekennzeichnet, daß sie neben üblichen Bestandteilen als Wirkstoffe Sericin und Pelargonsäure enthalten.

2. Haarkosmetische Mittel gemäß Anspruch 1, gekennzeichnet durch einen Anteil von 0,01 bis 1,0 Gew.-% Sericin und 0,01 bis 6,0 Gew.-% Pelargonsäure, jeweils bezogen auf die Gesamt-Zusammensetzung.

3. Haarkosmetische Mittel gemäß Anspruch 1, gekennzeichnet durch einen Anteil von 0,02 - 0,6 Gew.-% Sericin und 0,01 - 1,0 Gew.-% Pelargonsäure, jeweils bezogen auf die Gesamt-Zusammensetzung.

4. Haarkosmetische Mittel gemäß Anspruch 1, dadurch gekennzeichnet, daß sie einen pH-Wert kleiner 6 aufweisen.

5. Haarkosmetische Mittel gemäß Anspruch 1 in Form eines Haarwaschmittels auf wäßriger Basis, enthaltend waschaktive Verbindungen (Tenside), emulgierend wirkende Bestandteile, organische Säuren oder ein saures Puffersystem sowie ggf. Antioxydantien, Farb- und Konservierungsstoffe und Parfüm und weitere übliche Hilfsstoffe, dadurch gekennzeichnet, daß sie zusätzlich Sericin und Pelargonsäure enthalten.

6. Haarkosmetische Mittel gemäß Anspruch 1 in Form eines Haarkurmittels auf wäßriger Basis, enthaltend kationenaktive Verbindungen, emulgierend wirkende Bestandteile, hydrophobierende Substanzen, organische Säuren oder ein saures Puffersystem sowie ggf. Antioxydantien, Farb- und Konservierungsstoffe und Parfüm und weitere übliche Hilfsstoffe, dadurch gekennzeichnet daß sie zusätzlich Sericin und Pelargonsäure enthalten.

7. Verwendung eines Gemisches von Sericin und Pelargonsäure gemäß Anspruch 1 - 4 als pH-Wert und Schuppenstatus regulierender Wirkstoff in haarkosmetischen Mitteln auf wäßriger Basis.

**Claims**

1. Water-based cosmetic agents for hair, characterized in that, in addition to customary constituents, they contain sericin and pelargonic acid as active ingredients.

2. Cosmetic agents for hair according to claim 1, characterized by a content of 0.01 to 1.0% by weight of sericin and 0.01 to 6.0% by weight of pelargonic acid, in each case based on the total composition.

3. Cosmetic agents for hair according to claim 1, characterized by a content of 0.02 to 0.6% by weight of sericin and 0.01–1.0% by weight of pelargonic acid, in each case based on the total composition.

4. Cosmetic agents for hair according to claim 1, characterized in that they have a pH of less than 6.

5. Cosmetic agents for hair according to claim 1 in the form of a water-based shampoo containing detergent compounds (surfactants), constituents having an emulsifying action, organic acids or an acidic buffer system and, where appropriate, antioxidants, colorants and preservatives, and perfume and other customary auxiliaries, characterized in that they additionally contain sericin and pelargonic acid.

6. Cosmetic agents for hair according to claim 1 in the form of a water-based agent for hair health containing cationic compounds, constituents having an emulsifying action, water-repellent substances, organic acids or an acidic buffer system and, where appropriate, antioxidants, colorants and preservatives and perfume, and other customary auxiliaries, characterized in that they additionally contain sericin and pelargonic acid.

7. Use of a mixture of sericin and pelargonic acid according to claim 1 to 4 as active ingredient regulating the pH and scale status in water-based cosmetic agents for hair.

**Revendications**

1. Compositions cosmétiques pour les cheveux à base aqueuse, caractérisées en ce qu'elles contiennent comme principes actifs de la séricine et de l'acide pélargonique en plus de constituants habituels.

2. Compositions cosmétiques pour les cheveux suivant la revendication 1, caractérisées par une teneur de 0,01 à 1,0% en poids en séricine et de 0,01 à 6,0% en poids en acide pélargonique, chacune sur base de la composition totale.

3. Compositions cosmétiques pour les cheveux suivant la revendication 1, caractérisées par une teneur de 0,02 à 0,6% en poids en séricine et de 0,01 à 1,0% en poids en acide pélargonique, chacune sur base de la composition totale.

4. Compositions cosmétiques pour les cheveux suivant la revendication 1, caractérisées en ce qu'elles ont un pH inférieur à 6.

5. Compositions cosmétiques pour les cheveux suivant la revendication 1, sous la forme d'un shampooing à base aqueuse, contenant des composés tensioactifs (surfactifs), des constituants à effet émulsionnant, des acides organiques ou un système tampon acide, ainsi qu'éventuellement des antioxydants, des colorants et conservateurs et du parfum et d'autres substances auxiliaires habituelles, caractérisées en ce qu'elles contiennent de surcroît de la séricine et de l'acide pélargonique.

6. Compositions cosmétiques pour les cheveux suivant la revendication 1, sous la forme d'un agent de traitement de la chevelure à base aqueuse contenant des composés cationiques, des constituants à effet émulsionnant, des substances hydrophobes, des acides organiques ou un système tampon acide, ainsi qu'éventuellement des antioxydants, des colorants et conservateurs et du parfum et d'autres constituants auxiliaires habituels, caractérisées en ce qu'elles contiennent de surcroît de la séricine et de l'acide pélargonique.

7. Utilisation d'un mélange de séricine et d'acide pélargonique suivant les revendications 1 à 4 comme principe actif régulateur du pH et de l'état pelliculaire dans des compositions cosmétiques pour les cheveux à base aqueuse.